# EUROPÄISCHE PATENTSCHRIFT

(11) **EP 1 787 591 B1**
(45) Veröffentlichungstag und Bekanntmachung des Hinweises auf die Patenterteilung: **12.08.2009**
(21) Anmeldenummer: 05025324.4
(22) Anmeldetag: 21.11.2005
(51) Int. Cl.: A61B 17/04

(54) **Knochenanker**
Bone anchor
Ancre pour os

(43) Veröffentlichungstag der Anmeldung: 23.05.2007
(73) Patentinhaber: IST Innovative Shoulder Technology AG, 8808 Pfäffikon (CH)
(72) Erfinder: Kropf, Philip, 8808 Pfäffikon (CH); Leuzinger, Jan, 8808 Pfäffikon (CH)
(74) Vertreter: Blum, Rudolf Emil

(56) Entgegenhaltungen:
- WO-A-02/09601
- WO-A-02/11630
- WO-A-20/04062507

## Beschreibung

### Technisches Gebiet

Die Erfindung betrifft eine Vorrichtung zum Befestigen von Weichteilen an einem menschlichen oder tierischen Knochen.

### Stand der Technik

Bei verschiedenen human- und veterinärchirugischen Verfahren müssen Weichteile, beispielsweise Sehnen und Bänder, an einem Knochen fixiert werde. Das Fixieren kann erforderlich sein, wenn beispielsweise aufgrund eines Unfalls oder eines degenerativen Prozesses eine Sehne vom Knochen abgelöst ist, und die Sehne daran wieder zu fixieren ist. In der Chirugie dienen dazu verschiedenartige Vorrichtungen, die oft, wie auch in den folgenden Ausführungen, als Knochenanker bezeichnet werden. Der Knochenanker wird in einem vorbereitenden Schritt an einem freigelegten Knochen befestigt. Mit einem am Knochenanker fixierten oder geführten Nahtmaterial (z. B. Haltefaden) wird dann beispielsweise die Sehne am Knochenanker und damit am Knochen befestigt.

Ein bekannter Knochenanker ist der Statak^{®} Knochenanker der Firma Zimmer, Inc. Dieser Knochenanker hat einen zylinderförmigen Schraubenkörper mit einem Aussengewinde, eine distale kegelförmige Ankerspitze und einen proximalen Kopfteil. Das Aussengewinde erstreckt sich im wesentlichen zwischen der Ankerspitze und dem Kopfteil. Der Kopfteil hat eine Öse zur Aufnahme des Nahtmaterials senkrecht zur Längsachse des Schraubenkörpers. Der Kopfteil hat ausserdem eine mehrkantige äussere Oberfläche zum Ansetzen eines Werkzeugs. Der Kopfteil hat daher kein Aussengewinde.

Ein weiterer Knochenanker ist aus EP 829 233 A1 bekannt. Dieser Knochenanker hat einen zylinderförmigen Schraubenkörper, der in einen spitz zulaufenden distalen Teil übergeht. Ein Aussengewinde erstreckt sich entlang des Schraubenkörpers zwischen dem distalen Teil und einem proximalen Ende. Der Knochenanker hat senkrecht zur Längsachse und innerhalb des spitz zulaufenden Teils eine Durchbohrung zur Aufnahme von Nahtmaterial. In einem anderen Beispiel hat der Knochenanker anstatt der Durchbohrung eine vom distalen Teil ausgehende Kerbe zur Aufnahme von Nahtmaterial. In beiden Beispielen hat der Schraubenkörper zwei gegenüberliegende Nuten, die sich parallel zur Längsachse erstrecken und das Nahtmaterial von der Durchbohrung bzw. Kerbe zum proximalen Ende führen. Am proximalen Ende hat der Schraubenkörper einen Steckschlitz zum Ansetzen eines Werkzeuges.

Die genannten Knochenanker sind Schraubanker, die in den Knochen geschraubt werden. Neben diesen Schraubankern werden Einschlaganker verwendet, die mit einem speziellen Setzinstrument in den Knochen eingeschlagen werden. Vom zylinderförmigen Körper des Knochenankers abstehende Flügel oder Widerhaken verkeilen sich unter dem kortikalen Knochen und sollen so den Knochenanker sicher im Knochen halten. Am proximalen Ende hat der Knochenanker quer zu seiner Längsachse eine Durchführung zur Aufnahme eines Haltefadens. Ein Beispiel für einen solchen Knochenanker ist der GII^{®} Anker von DePuy Mitek, Inc.

Mediziner, vor allem Chirurgen, erwarten von Knochenankern, dass sie zuverlässig implantierbar und praktisch handhabbar sind, z. B. dass der Haltefaden, wenn nich bereits herstellerseitig vorhanden, leicht einfädelbar ist, nicht ungewollt abreisst und sich nicht verheddert. Hinzukommt, dass Knochenanker einen sicheren Halt im Knochen gewähren müssen.

Beim Statak^{®} Knochenanker hat der Kopfteil eine Doppelfunktion, er dient nämlich sowohl zur Halterung des Nahtmaterials als auch als Ansatz für das Werkzeug. Wegen des relativ geringen Querschnitts des Kopfteils besteht die Gefahr, dass der Kopfteil und damit auch die Fadenhalterung während dem Einsetzen des Knochenankers abgedreht wird. Bei dem aus EP 829 233 A1 bekannten Knochenanker ist zwar der Steckschlitz im Schraubenkörper versenkt, so dass die Gefahr des Abdrehens nicht besteht, die Notwendigkeit, den Faden in den Nuten von der Spitze zum proximalen Ende zu führen, erscheint jedoch wenig praktikabel. Da der GII^{®} Anker nicht geschraubt wird, besteht bei diesem Anker das Problem das Abdrehens nicht. Bei diesem Anker kann es jedoch von Nachteil sein, dass der gesetzte Anker wegen der Verkeilung der Widerhaken nicht, oder nur schwer wieder zu entfernen ist.

Ferner ist aus der Patentanmeldung WO 02/09601 A2 eine medizinische Schraube bekannt, die einen ersten, mit einem Gewinde versehenen Abschnitt und einen sich an diesen ersten Abschnitt anschliessenden, gewindefreien Abschnitt aufweist. Am ersten Abschnitt ist ein Kopf vorgesehen, der eine Öse aufweist, durch die ein chirurgischer Faden geführt werden kann.

Aus der Patentanmeldung WO 2004/062507 A2 ist eine Befestigungsvorrichtung bekannt, die als Anker in einem Knochen verwendet werden kann und die einen mit einem Gewinde versehenen Bereich und einen sich an diesen Bereich anschliessenden Bereich, der einen Schaft und eine Spitze am Ende des Schaftes aufweist, umfasst. Die Befestigungsvorrichtung weist in dem mit dem Gewinde versehenen Bereich eine querlaufende Bohrung auf, durch die ein chirurgischer Faden geführt werden kann.

In der Patentanmeldung WO 02/11630 A1 ist ein Knochenverankerungssystem beschrieben, welches eine Knochenschraube als ersten Bereich und eine selbstschneidende Spitze als zweiten Bereich umfasst. Am proximalen Ende der Knochenschraube ist eine Öffnung vorgesehen, die einen chirurgischen Faden aufnehmen kann.

### Darstellung der Erfindung

Es besteht der Bedarf an einem Knochenanker der eingangs genannten Art mit verbesserten Eigenschaften hinsichtlich Zuverlässigkeit, Handhabbarkeit und Verankerungssicherheit im Knochen.

Demgemäss hat eine Vorrichtung zum Befestigen von Weichteilen an einem Knochen, der eine Kortikalis vorgegebener Dicke hat, in einem Ausführungsbeispiel einen Körper mit einem proximalen Ende und einem distalen Ende, der einen vom proximalen Ende ausgehenden ersten Abschnitt mit einem Aussengewinde und einen daran anschliessenden gewindefreien zweiten Abschnitt aufweist. Der erste Abschnitt hat eine Länge, die der Dicke der Kortikalis des Knochens angepasst ist. Die Länge des ersten Abschnitts hat einen Wert, der einer bei einem Menschen typischerweise vorkommanden Dicke einer Kortikalis entspricht. Diese Vorrichtung hat ausserdem am proximalen Ende eine Vorrichtung, die sich vom proximalen Ende aus in den Körper hinein erstreckt und zur Aufnahme eines Haltefadens dient.

Wie aus der folgenden Beschreibung und den Zeichnungen zu entnehmen ist, kann der hier beschriebene Knochenanker als ein Einschlaganker aufgefasst werden, der in der Nähe des proximalen Endes ein Aussengewinde hat. Dadurch kann der Knochenanker sehr schnell in den Knochen eingesetzt werden. Das Aussengewinde fixiert dabei den Knochenanker in der Kortikalis. Trotzdem lässt er sich bei Bedarf relativ einfach wieder entfernen.

Die am proximalen Ende vorhandene Vorrichtung ist so ausgestaltet, dass sie ein erleichtertes Einfädeln des Haltefadens ermöglicht, und dass keine Kanten, an denen der Haltefaden evtl. abreissen könnte, vorhanden sind. Die Vorrichtung schliesst mit dem proximalen Ende des Knochenankers ab, wodurch der implantierte Knochenanker im wesentlichen mit der äusseren Oberfläche des Knochens abschliesst. Dadurch wird vermieden, dass der Haltefaden über eine Kante des Knochens geführt wird und daran abreissen könnte.

### Kurze Beschreibung der Zeichnungen

Weitere Ausgestaltungen, Vorteile, neue Eigenschaften und Anwendungen der Erfindung ergeben sich aus der folgenden detaillierten Beschreibung unter Einbezug der Zeichnungen. In den Zeichnungen haben gleiche Elemente die gleichen Bezugszeichen. In den Zeichnungen zeigen:
Figur 1 eine schematische Seitenansicht eines Ausführungsbeispiels eines Knochenankers,
Figur 2 einen schematischen Schnitt durch den in Fig. 1 gezeigten Knochenanker entlang einer Längsachse,
Figur 3 eine Draufsicht auf den in Fig.1 gezeigten Knochenanker, und
Figuren 4 und 5 schematische Seitenansichten von weiteren Ausführungsbeispielen eines Knochenankers.

### Wege zur Ausführung der Erfindung

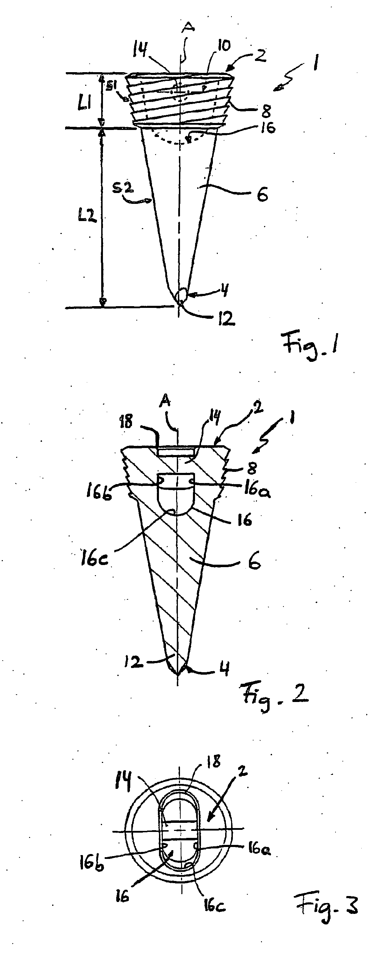

Figur 1 zeigt ein Ausführungsbeispiel eines Knochenankers 1, der zur Befestigung von Weichteilen an einem Knochen dient. Der Knochenanker 1 hat einen Körper 6, der sich symmetrisch um eine Längsachse A von einem proximalen Ende 2 zu einem distalen Ende 4 erstreckt. Der Körper 6 hat einen Abschnitt S1 einer Länge L1, der sich vom proximalen Ende 2 ausgehend in Richtung des distalen Endes 4 erstreckt. Der Abschnitt S1 hat ein Aussengewinde 8. Der Körper 6 hat ausserdem einen Abschnitt S2 einer Länge L2, der an den Abschnitt S1 anschliesst und sich bis zum distalen Ende 4 erstreckt. Der Abschnitt S2 ist gewindefrei.

Wie weiter unten ausgeführt, ist die Länge L1 des Abschnitts S1 so gewählt, dass das Aussengewinde 8 in der Kortikalis des Knochens Halt findet. In einem Ausführungsbeispiel beträgt die Länge L1 etwa 3 mm, und die Länge L2 etwa 6 mm. Die Werte für die Länge L1 können variieren, sie entsprechen aber der beim Menschen typischerweise vorkommenden Dicke der Kortikalis. Die Gesamtlänge des Knochenankers 1 kann etwa 5 bis etwa 10 mm betragen.

Im in Figur 1 gezeigten Ausführungsbeispiel verjüngt sich der Körper 6 vom proximalen Ende 2 in Richtung des distalen Endes 4. Der Körper 6 kann beispielsweise kegelförmig oder kegelstumpfförmig sein. Eine sich verjüngende oder konische Form kann für das Einschlagen in den Knochen vorteilhaft sein. Es versteht sich jedoch, dass der Knochenanker 1 in einem anderen Ausführungsbeispiel eine andere Form haben kann, z. B. die Form eines Zylinders.

Am distalen Ende 4 hat der Knochenanker 1 eine mit dem Körper 6 verbundene Spitze 12. Die Spitze 12 und der Körper 6 sind in einem Ausführungsbeispiel aus einem Stück Material geformt. Das Material ist biokompatibel und kann beispielsweise Edelstahl, Titan, eine Titanlegierung oder ein resorbierbarer Werstoff (z. B. Polilaktid oder Poliglykonat) sein.

Der Knochenanker 1 hat ausserdem eine Vorrichtung 10, die sich vom proximalen Ende 2 aus in den Körper 6 hinein erstreckt und zur Aufnahme eines Haltefadens dient. In Figur 1 ist die Vorrichtung 10 zur Veranschaulichung durch gestrichelte Linien angedeutet, da sie in der Seitenansicht des Knochenankers 1 nicht sichtbar ist. Die Vorrichtung 10 bildet bei dem gezeigten Knochenanker 1 im Bereich des proximalen Endes 2 einen Hohlraum, der durch eine Wandfläche 16 begrenzt und am proximalen Ende 2 offen ist. Die Wandfläche 16 hat in Figur 1 eine konkave Form. Die Vorrichtung 10 hat ausserdem einen Steg 14 mit einer kreisförmigen Querschnittsfläche, der sich im Hohlraum erstreckt.

Figur 2 zeigt einen schematischen Schnitt durch den in Figur 1 gezeigten Knochenanker 1 entlang der Längsachse A. In dem in Figur 2 gezeigten Ausführungsbeispiel hat die Wandfläche 16 einen gekrümmten Flächenabschnitt 16c und zwei Seitenflächen 16a, 16b, die sich parallel zur Längsachse A erstrecken und in den Flächenabschnitt 16c übergehen. Die Wandfläche 16 formt den Hohlraum im gezeigten Ausführungsbeispiel so, dass ein scheibenförmiger Hohlraum entsteht. Es versteht sich, dass in anderen Ausführungsbeispielen der Hohlraum eine andere Form, z. B. die Form eines Halbovals oder einer Halbkugel, annehmen kann.

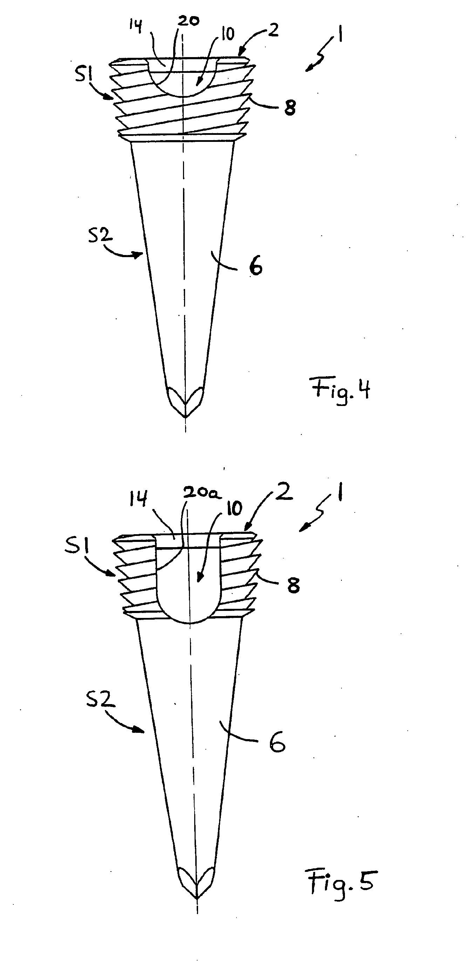

Der Steg 14 erstreckt sich im wesentlichen quer zur Längsachse A zwischen den Seitenteilen 16a, 16b. Dabei befindet sich der Steg 14 in einem Ausführungsbeispiel auf dem Niveau der Ebene des proximalen Endes 2, wie beispielsweise in den Figuren 4 und 5 gezeigt. In der in Figur 2 gezeigten Darstellung ist der Steg 14 von der Ebene des proximalen Endes 2 in Richtung des Hohlraumes abgesetzt. In einem Ausführungsbeispiel ist der Steg 14 weniger als etwa 1 mm vom proximalen Ende 2 abgesetzt. Der Übergang von der Ebene des proximalen Endes 2 in den Hohlraum ist innerhalb eines Bereichs 18 abgerundet oder abgeschrägt, um Kanten zu vermeiden, an denen der Haltefaden abgetrennt werden könnte.

Figur 3 zeigt eine Draufsicht auf den in Figur 1 gezeigten Knochenanker 1. Sichtbar sind das proximale Ende 2, der durch die Wandfläche 16 gebildete Hohlraum und der sich im Hohlraum erstreckende Steg 14. In der Draufsicht hat der Hohlraum eine langgezogene ovale Öffnung, die durch die Seitenflächen 16a, 16b und den Flächenabschnitt 16c gebildet wird. Der Steg 14 teilt die ovale Öffnung in zwei etwa gleich grosse Teilöffnungen. Ein Chirurg kann beispielsweise einen Haftfaden durch eine Teilöffnung in den Hohlraum und um den Steg 14 herum führen, und aus der anderen Teilöffnung entnehmen.

Am proximalen Ende 2 kann der Chirurg auch ein Werkzeug ansetzen, um den Knochenanker 1 in den Knochen zu schrauben. In einem Ausführungsbeispiel ist das Werkzeug so ausgestaltet, dass es in den Hohlraum greift. Das Werkzeug kann eine dem Durchmesser des Steges 14 entsprechende Aussparung haben, um das Werkzeug über den Steg 14 hinweg in den Hohlraum zu führen, so dass die Drehkräfte im Innern des Körpers 6 greifen können. Die Gefahr der Beschädigung des Knochenankers 1 ist dadurch wesentlich reduziert.

Die Figuren 4 und 5 zeigen schematische Seitenansichten von weiteren Ausführungsbeispielen eines Knochenankers 1. Bei diesen Ausführungsbeispielen ist in einem oder mehreren Bereichen des Abschnitts S1 kein Ankermaterial und damit auch kein Aussengewinde 8 vorhanden. Von dem in den Figuren 1 - 3 gezeigten Knochenanker 1 kann beispielsweise in einem Bereich Ankermaterial über eine Länge von mehreren Gewindegängen entfernt (z. B. abgeschliffen) werden. Dadurch ist in der Nähe des proximalen Endes 2 weniger das Knochenmaterial verdrängendes Ankermaterial vorhanden. Das Knochenmaterial kann sich daher in diesen Bereich entspannen.

Bei dem in Figur 4 gezeigten Ausführungsbeispiel des Knochenankers 1 ist in einem Bereich 20 das Ankermaterial vom proximalen Ende 2 ausgehend über etwa zwei Gewindegänge hinweg entfernt, so dass eine seitliche Öffnung entsteht, durch die der Steg 14 und ein Teil des durch die Wandfläche 16 gebildeten Hohlraumes sichtbar sind. Auf einer gegenüberliegenden Seite der Öffnung kann das Ankermaterial auf die gleiche Weise ebenfalls entfernt sein, wodurch eine weitere seitliche Öffnung entsteht.

Bei dem in Figur 5 gezeigten Ausführungsbeispiel des Knochenankers 1 ist dagegen in einem Bereich 20a das Ankermaterial über die gesamte Länge des Abschnitts S1 hinweg entfernt. Der Steg 14 und ein Teil des durch die Wandfläche 16 gebildeten Hohlraumes sind dabei ebenfalls durch eine gebildete seitliche Öffnung sichtbar. Auch bei diesem Ausführungsbeispiel kann auf einer gegenüberliegenden Seite der Öffnung das Ankermaterial auf die gleiche Weise entfernt sein, wodurch eine weitere seitliche Öffnung entsteht.

Der im vorhergehenden durch Ausführungsbeispiele beschriebene Knochenanker 1 ermöglicht ein praktisches und zuverlässiges Hantieren, da die den Haltefaden aufnehmende und führende Vorrichtung 10 am proximalen Ende 2 für den Chirurgen leicht zugänglich ist und im implantierten Zustand im wesentlichen mit der Knochenoberfläche abschliesst. Der eingefädelte Haltefaden wird bei diesen Ausführungsbeispielen nicht über ein Knochenkante geführt, an der er abreissen könnte. Die konkave Wandfläche 16 unterstützt den Chirurgen beim Einfädeln des Haltefadens. Die in den Figuren 4 und 5 gezeigten seitlichen Öffnungen erleichtern zusätzlich das Einfädeln des Haltefadens. Die Vorrichtung 10 und insbesondere der Steg 14 sind so ausgestaltet, dass sie keine scharfen Kanten aufweisen, an denen der Haltfaden reissen könnte.

Der Knochenanker 1 gewährt ausserdem einen sicheren Halt im Knochen. Dies wird vor allem dadurch erreicht, dass sich das Aussengewinde 8 im Bereich des proximalen Endes 2 entlang des Abschnitts S1 erstreckt. Das Aussengewinde 8 findet damit in der Kortikalis des Knochens Halt und nicht im Bereich des Knochenmarkes, in dem der gewindefreie Abschnitt S2 sitzt. Bei Patienten, bei denen im Bereich des Knochenmarkes wenig Struktur vorhanden ist, beispielsweise bei älteren Patienten, kann dadurch ein sicherer Halt im Knochen erzielt werden.

Der im vorhergehenden durch Ausführungsbeispiele beschriebene Knochenanker 1 kann direkt in den Knochen eingeschlagen werden. Erst dann wird der Knochenanker 1 durch Schrauben weiter in den Knochen geschoben, wobei der Abschnitt S1 im wesentlichen in der Kortikalis zum Liegen kommt.

Je nach Ausführungsbeispiel muss sich das Aussengewinde 8 nicht bis zum äussersten Rand des proximalen Endes 2 erstrecken, obschon dies bevorzugt ist; es erstreckt sich aber so nahe zum proximalen Ende 2, dass die Funktion der Verankerung durch das Aussengewinde 8 in der Kortikalis erfüllt werden kann.

### Bezugszeichenliste

- A: Achse
- S1: Abschnitt (Länge L1)
- S2: Abschnitt (Länge L2)
- 1: Knochenanker
- 2: proximales Ende
- 4: distales Ende
- 6: Körper
- 8: Aussengewinde
- 10: Vorrichtung
- 12: Spitze
- 14: Steg
- 16: Wandfläche
- 16a: Seitenfläche
- 16b: Seitenfläche
- 16c: Flächenabschnitt
- 18: Bereich
- 20: Bereich
- 20a: Bereich

## Patentansprüche

1. Knochenanker (1) zum Befestigen von Weichteilen an einem Knochen, der eine Kortikalis vorgegebener Dicke hat,
der einen Körper (6) mit einem proximalen Ende (2) und einem distalen Ende (4) hat, der einen vom proximalen Ende (2) ausgehenden ersten Abschnitt (S1) mit einem Aussengewinde (8) und einen daran anschliessenden gewindefreien zweiten Abschnitt (S2) aufweist und
der eine Vorrichtung (10) am proximalen Ende (2) hat, die sich vom proximalen Ende (2) aus in den Körper (6) hinein erstreckt und zur Aufnahme eines Haltefadens dient,
**dadurch gekennzeichnet, dass** der erste Abschnitt (S1) des Körpers (6), der geeignet ist, in der Kortikalis des menschlichen'Körpers Halt zu finden, eine Länge (L1) hat, die 3 bis 4 mm beträgt, so dass der Wert einer bei einem Menschen typischerweise vorkommenden Dicke einer Kortikalis entspricht.

2. Knochenanker nach Anspruch 1, bei dem eine Spitze (12) am distalen Ende (4) des Körpers (6) vorhanden ist.

3. Knochenanker nach einem der vorangehenden Ansprüche, bei dem die Vorrichtung (10) innerhalb des Körpers (6) einen Steg (14) hat, der die Vorrichtung (10) in einen ersten und einen zweiten Teil trennt, wobei der Haltefaden vom ersten Teil zum -zweiten Teil durchführbar ist.

4. Knochenanker nach Anspruch 3, bei dem Steg (14) einen kreisförmigen Querschnitt hat.

5. Knochenanker nach einem der Ansprüche 3 bis 4, bei dem Steg (14) nahe des proximalen Endes (2) angeordnet ist.

6. Knochenanker nach einem der vorangehenden Ansprüche, bei dem die Vorrichtung (10) im Körper (6) eine konkave Wandfläche (16) hat.

7. Knochenanker nach einem der vorangehenden Ansprüche, bei dem ein Übergang von einer Ebene des proximalen Endes (2) in Richtung der Vorrichtung (10) innerhalb eines Bereichs (18) abgerundet oder abgeschrägt ist.

8. Knochenanker nach einem der vorangehenden Ansprüche, bei dem sich der Körper (6) vom proximalen Ende (2) in Richtung des distalen Endes (4) verjüngt.

9. Knochenanker nach einem der vorangehenden Ansprüche, bei dem vom ersten Abschnitt (S1) in mindestens einem Bereich (20, 20a) Ankermaterial entfernt ist, so dass sich eine seitliche Öffnung ergibt.

## Claims

1. Bone anchor (1) for attaching soft tissues to a bone, having a corticalis of predetermined thickness,
which has a body (6) with a proximal end (2) and a distal end (4), which has a first section (S1) with an outside thread (8) starting from the proximal end (2) and a thread-free second section (S2) following the first section and
which has a device (10) at the proximal end (2), that extends from the proximal end (2) into the body (6) and serves to hold a holding thread,
**characterized in that** the first section (S1) of the body (6), which is suitable to hold on to the corticalis of the human body, has a length (L1) of 3 to 4 mm, so that the value corresponds to the thickness, that typically exists in humans, of a corticalis.

2. Bone anchor according to claim 1, in which there is a tip (12) at the distal end (4) of the body (6).

3. Bone anchor according to one of the preceding claims, in which the device (10) within the body (6) has a bar (14) that separates the device (10) into a first and a second part, wherein the holding thread can be threaded from the first part to the second part.

4. Bone anchor according to claim 3, in which the bar (14) has a circular cross-section.

5. Bone anchor according to one of the claims 3 to 4, in which the bar (14) is arranged near the proximal end (2).

6. Bone anchor according to one of the preceding claims, in which the device (10) has a concave wall area (6) in the body (6).

7. Bone anchor according to one of the preceding claims, in which a transition of a plane of the proximal end (2) in the direction of the device (10) is rounded or inclined within a region (18).

8. Bone anchor according to one of the preceding claims, in which the body (6) tapers from the proximal end (2) in the direction of the distal end (4).

9. Bone anchor according to one of the preceding claims, in which anchor material is removed from the first section (S1) in at least one region (20, 20a) such that a side opening is produced.

## Revendications

1. Ancre pour os (1) pour fixer des parties molles sur un os, qui présente des parois corticales d'une épaisseur donnée,
qui a un corps (6) avec une extrémité proximale (2) et une extrémité distale (4), qui présente une première section (S1) partant de l'extrémité proximale (2) et pourvue d'un filetage (8) et une seconde section (S2) non filetée s'y raccordant et
qui a un dispositif (10) à l'extrémité proximale (2) qui s'étend à partir de l'extrémité proximale (2) dans le corps (6) et sert à recevoir un filament de maintien,
**caractérisée en ce que** la première section (S1) du corps (6) qui est capable de s'attacher dans les parois corticales du corps humain a une longueur (L1) de 3 à 4 mm, de sorte que la valeur correspond à l'épaisseur de parois corticales présentes typiquement dans un humain.

2. Ancre pour os selon la revendication 1, présentant une pointe (12) à l'extrémité distale (4) du corps (6).

3. Ancre pour os selon l'une des revendications précédentes, dans laquelle le dispositif (10) à l'intérieur du corps possède une cloison (14) qui sépare le dispositif (10) en une première et une seconde partie, le filament de maintien pouvant être amené de la première à la seconde partie.

4. Ancre pour os selon la revendication 3, dans laquelle la cloison (14) a une section circulaire.

5. Ancre pour os selon l'une des revendications 3 à 4, dans laquelle la cloison (14) est disposée près de l'extrémité proximale (2).

6. Ancre pour os selon l'une des revendications précédentes, dans laquelle le dispositif (10) dans le corps (6) est une surface de paroi (16) concave.

7. Ancre pour os selon l'une des revendications précédentes, dans laquelle une transition depuis un plan de l'extrémité proximale (2) en direction du dispositif (10) est arrondie ou chanfreinée à l'intérieur d'une zone (18).

8. Ancre pour os selon l'une des revendications précédentes, dans laquelle le corps (6) se rétrécie depuis l'extrémité proximale (2) vers l'extrémité distale (4).

9. Ancre pour os selon l'une des revendications précédentes, dans laquelle dans au moins une zone (20, 20a) de la première section (S1) de la matière de l'ancre est enlevée, de sorte que l'on obtient une ouverture latérale.
